# EUROPEAN PATENT SPECIFICATION

(11) **EP 4 395 898 B1**
(45) Date of publication and mention of the grant of the patent: **04.11.2026**
(21) Application number: 22772926.6
(22) Date of filing: 01.09.2022
(51) Int. Cl.: A61P 35/00, C07D 493/20, A61K 31/35

(54) **NITROGEN-CONTAINING DERIVATIVES OF NARASIN, SYNTHESIS AND USES THEREOF**
STICKSTOFFHALTIGE DERIVATE VON NARASIN, SYNTHESE UND VERWENDUNGEN DAVON
DÉRIVÉS DE NARASIN CONTENANT DE L'AZOTE, LEUR SYNTHÈSE ET LEURS UTILISATIONS

(30) Priority: 03.09.2021 EP 21306209
(43) Date of publication of application: 10.07.2024
(73) Proprietor: Centre National de la Recherche Scientifique, 75016 Paris (FR); INSTITUT NATIONAL DE LA SANTE ET DE LA RECHERCHE MEDICALE (INSERM), 75013 Paris (FR); Institut Curie, 75005 Paris (FR)
(72) Inventor: RODRIGUEZ, Raphaël, 30210 Vers-Pont-du-Gard (FR); CAÑEQUE, Tatiana, 75004 Paris (FR); MÜLLER, Sebastian, 94200 Ivry sur Seine (FR); ANTOSZCZAK, Michal, 62-003 Biedrusko (PL)
(74) Representative: Novagraaf Technologies
(86) International application number: PCT/EP2022/074390
(87) International publication number: WO 2023/031362

(56) References cited:
- ANTOSZCZAK MICHAL ET AL: "Iron-Sensitive Prodrugs That Trigger Active Ferroptosis in Drug-Tolerant Pancreatic Cancer Cells", JOURNAL OF THE AMERICAN CHEMICAL SOCIETY, vol. 144, no. 26, 13 June 2022 (2022-06-13), pages 11536 - 11545, XP093270352, ISSN: 0002-7863, Retrieved from the Internet <URL:https://pubs.acs.org/doi/pdf/10.1021/jacs.2c03973> DOI: 10.1021/jacs.2c03973
- CHEN JING ET AL: "Narasin inhibits tumor metastasis and growth of ER[alpha]-positive breast cancer cells by inactivation of the TGF-[beta]/SMAD3 and IL-6/STAT3 signaling pathways", MOLECULAR MEDICINE REPORTS, vol. 22, no. 6, 21 October 2020 (2020-10-21), GR, pages 5113 - 5124, XP055871278, ISSN: 1791-2997, DOI: 10.3892/mmr.2020.11624
- MILLER S C ET AL: "Identification of known drugs that act as inhibitors of NF-@?B signaling and their mechanism of action", BIOCHEMICAL PHARMACOLOGY, ELSEVIER, US, vol. 79, no. 9, 1 May 2010 (2010-05-01), pages 1272 - 1280, XP026939405, ISSN: 0006-2952, [retrieved on 20100306], DOI: 10.1016/J.BCP.2009.12.021

## Description

The present invention concerns novel nitrogen-containing derivatives of narasin, their synthesis and their uses in therapy, notably for the treatment of cancer.

Resistance to chemotherapy and radiotherapy remains a major obstacle in the successful treatment of cancer. Resistance may occur during cancer treatment because of many reasons, such as some of the cancer cells which are not killed can mutate and become resistant, gene amplification resulting in the overexpression of a protein that renders the treatment ineffective may occur, or cancer cells may develop a mechanism to inactivate the treatment.

Narasin is a promising antiproliferative drug candidate in the treatment of cancer, in particular for the treatment of drug-resistant cancer cell lines. However, this molecule contains a carboxyl radical which is responsible for its degradation in the cells.

Therefore, there is a need for the development of novel and efficient anti-cancer drugs, which would be efficient while being specific for tumoral tissues. Especially, there is a need for novel and efficient anti-cancer drugs able to kill both cancer stem cells and therapy-resistant cancer cells.

There is also a need for new molecules able to target the lysosomal iron, and also inducing the cell death of therapy-resistant cancer cells.

The aim of the present invention is thus to provide narasin derivatives able to target the lysosomal iron and also able to induce the cell death of therapy-resistant cancer cells.

Another aim of the present invention is to provide narasin derivatives with anti-cancer activity, and preferably being able to kill both cancer stem cells and therapy-resistant cancer cells Narasin derivatives with anticancer properties have been disclosed in CHEN JING ET AL: "Narasin inhibits tumor metastasis and growth of ERα-positive breast cancer cells by inactivation of the TGF-β/SMAD3 and IL-6/STAT3 signaling pathways", MOLECULAR MEDICINE REPORTS, vol. 22, no. 6, 21 October 2020, pages 5113-5124 and in MILLER S C ET AL: "Identification of known drugs that act as inhibitors of NF-κB signaling and their mechanism of action", BIOCHEMICAL PHARMACOLOGY, ELSEVIER, US, vol. 79, no. 9, 1 May 2010, pages 1272-1280. .

Therefore, the present invention relates to a compound having the formula (I): 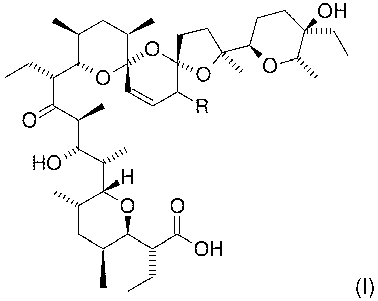
wherein R is a group -NR¹R², R¹ and R² being identical or different, preferably different, and independently chosen from the group consisting of: H, a (C₁-C₂₀)alkyl group, a (C₃-C₆)cycloalkyl group, a (C₂-C₂₀)alkynyl group, and a (C₆-C₁₀)aryl(C₁-C₆)alkyl group,
as well as its epimers.

Preferably, R¹ and R² are not simultaneously H.

According to the invention, the term "epimers" refers to a specific type of stereoisomers that have multiple asym metric carbons, but only differ from one another by the configuration at one of the asymmetric carbons.

In the compounds of formula (I), the bond carrying the R group may be an up or down bond. In formula (I), the bond -R corresponds to one of the following bonds:

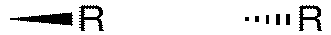

The present invention also relates to a compound having the formula (I-1) or (I-2):

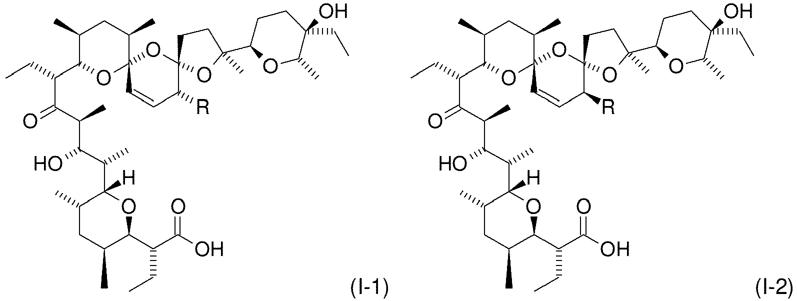

R being as defined above in formula (I).

The following definitions are set forth to illustrate and define the meaning and scope of the various terms used to describe the invention herein.

The expression "Cₜ-C_{z}" means a carbon-based chain which can have from t to z carbon atoms, for example C₁-C₃ means a carbon-based chain which can have from 1 to 3 carbon atoms.

The term "alkyl group" means: a linear or branched, saturated, hydrocarbon-based aliphatic group comprising, unless otherwise mentioned, from 1 to 20 carbon atoms, preferably from 1 to 15, and more preferably from 3 to 10, or 1 to 6, carbon atoms. By way of examples, mention may be made of methyl, ethyl, n-propyl, isopropyl, n-butyl, isobutyl, tert-butyl, n-pentyl, isopentyl, n-hexyl, octyl or dodecyl groups, and preferably ethyl, n-propyl, n-butyl, isobutyl, n-pentyl, n-hexyl or dodecyl groups.

The term "aryl group" means: a cyclic aromatic group comprising between 6 and 10 carbon atoms. By way of examples of aryl groups, mention may be made of phenyl or naphthyl groups.

The term "cycloalkyl group" means: a cyclic carbon-based group comprising, unless otherwise mentioned, from 3 to 6 carbon atoms. By way of examples, mention may be made of cyclopropyl, cyclobutyl, cyclopentyl, cyclohexyl, etc. groups. Preferably, the (C₃-C₆)cycloalkyl is cyclopropyl.

The term "alkynyl" as employed herein includes unsaturated, nonaromatic, hydrocarbon groups having 2 to 20 carbons, preferably 2 to 6 carbons, and comprising at least one triple bond. Preferably, the alkyne group is linear. Preferably, the alkynyl group is a -(CH₂)ₙ-C≡CH group, n being an integer comprised from 1 to 4.

Preferably, the alkynyl group comprises 2 to 10 carbon atoms, preferably 2 to 6 carbon atoms. Preferably, the alkynyl group comprises 3 carbon atoms. Preferably, the alkynyl is propynyl.

When an alkyl radical is substituted with an aryl group, the term "arylalkyl" or "aralkyl" radical is used. The "arylalkyl" or "aralkyl" radicals are aryl-alkyl-radicals, the aryl and alkyl groups being as defined above. Among the arylalkyl radicals, mention may in particular be made of the benzyl or phenethyl radicals.

Preferably, the arylalkyl according to the invention is a group -(CH₂)ₙ-aryl, wherein n is an integer from 1 to 5, and aryl is as defined above.

The aryl may be substituted by at least one substituent chosen from the group consisting of: a hydroxyl group, a (C₁-C₂₀)alkyl group, preferably a (C₁-C₆)alkyl group, and a halogen. The term "halogen" means: a fluorine, a chlorine, a bromine or an iodine.

Preferably, the aryl is substituted, preferably in para position, by at least one group chosen from a hydroxyl group, a (C₁-C₆)alkyl group such as a methyl, and a halogen such as F or Cl. Preferably, the arylalkyl group is a benzyl which is not substituted, or a substituted benzyl by at least one group, preferably in para position, chosen from a hydroxyl group, a (C₁-C₆)alkyl group such as a methyl, and a halogen such as F or Cl.

According to an embodiment, in formula (I), (I-1) or (I-2), R is a group -NR¹R², where R¹ is selected from the group consisting of: a (C₁-C₂₀)alkyl group, a (C₃-C₆)cycloalkyl group, a (C₂-C₂₀)alkynyl group, and a (C₆-C₁₀)aryl(C₁-C₆)alkyl group, and R² is chosen from a (C₁-C₂₀)alkyl group and a (C₂-C₂₀)alkynyl group.

According to an embodiment, in formula (I), (I-1) or (I-2), R is a group -NR¹R², where R¹ and R² are different and independently chosen from H, a (C₃-C₆)cycloalkyl group, and a (C₂-C₂₀)alkynyl group.

According to an embodiment, in formula (I), (I-1) or (I-2), R is -NR¹R². In this case, preferably R² is H and R¹ is a (C₃-C₆)cycloalkyl group or a (C₂-C₂₀)alkynyl group, preferably a (C₂-C₆)alkynyl group. Preferably, R² is H and R¹ is cyclopropyl or propynyl.

According to an embodiment, in formula (I), (I-1) or (I-2), R is a group -NHR², R² being selected from the group consisting of: a (C₁-C₂₀)alkyl group, a (C₃-C₆)cycloalkyl group, a (C₂-C₂₀)alkynyl group, and a (C₆-C₁₀)aryl(C₁-C₆)alkyl group.

Preferably, R² is a (C₃-C₆)cycloalkyl group or a (C₂-C₂₀)alkynyl group.

More preferably, R² is a cyclopropyl or a propynyl group.

The present invention also relates to a compound as defined above, having the formula (I-3):

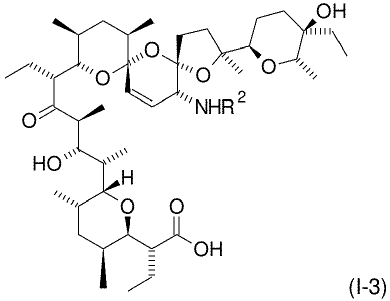

R² being as defined above.

Preferably, in formula (I-3), R² is a (C₃-C₆)cycloalkyl group or a (C₂-C₂₀)alkynyl group.

As preferred compounds according to the present invention, the following compounds may be mentioned:

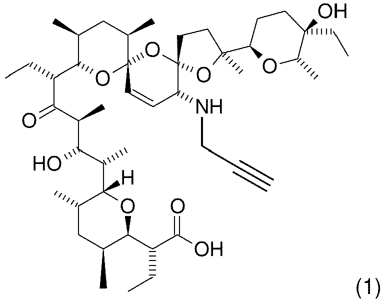

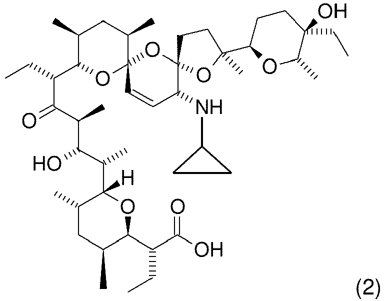

### Preparation of the compounds of the invention

The present invention also relates to process for the preparation of a compound of the invention, having the formula (I) as defined above, comprising the following steps:
- a step of oxidizing the C20 allylic hydroxyl of narasin to the corresponding α,β-unsaturated ketone; and
- a step of reacting the α,β-unsaturated ketone with an amine HNR¹R², in order to obtain the corresponding imine, and a step for simultaneously or subsequently reducing the imine, in order to obtain the corresponding C20-amine derivatives of narasin.

The present invention also relates to process for the preparation of a compound of the invention, having the formula (I) as defined above, comprising the following steps:
- a step of oxidizing the C20 allylic hydroxyl of narasin to the corresponding α,β-unsaturated ketone;
- a step of protecting the C1 carboxyl of the α,β-unsaturated ketone of narasin, in order to obtain a protected α,β-unsaturated ketone of narasin;
- a step of reacting the α,β-unsaturated ketone with an amine HNR¹R², in order to obtain the corresponding imine, and a step for simultaneously or subsequently reducing the imine, in order to obtain the corresponding C20-amine derivatives of narasin; and
- a step of removing the masking group at the C1 position of the protected C20-amine derivatives of narasin to obtain the compound of formula (I).

According to an embodiment, the process according to the invention comprises the chemoselective oxidation of the C20 allylic hydroxyl of narasin followed by the stereoselective reductive amination of C20-oxonarasin.

According to an embodiment, the process according to the invention comprises the chemoselective oxidation of the C20 allylic hydroxyl of narasin, then the blocking of the C1 functionality, followed by the stereoselective reductive amination of C20-oxonarasin and then the unblocking of the C1 functionality.

The compounds of the invention may be prepared by the following process, which is illustrated in the examples.

As mentioned above, the -NR¹R² group can be introduced by the following method:
(a) oxidizing the C20 allylic hydroxyl of narasin to the α,β-unsaturated ketone.

Methods for the chemoselective oxidation of allylic alcohols are well known in the art. Advantageously, oxidation of the C20 hydroxyl of narasin is quantitatively performed with activated MnO₂.

The C20 allylic hydroxyl of narasin may be represented by the following formula (A): 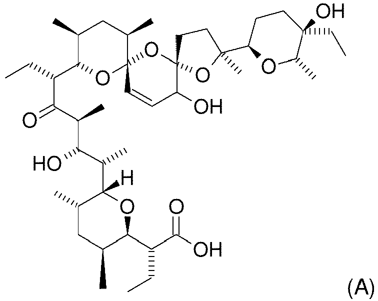 and preferably by the following formula (A-1):

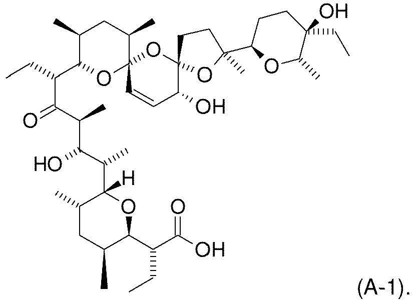

The α,β-unsaturated ketone of narasin may be represented by the following formula (B):

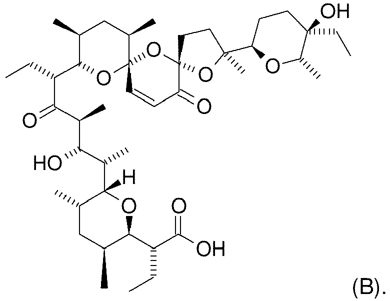

(b) Optionally protecting the C1 carboxyl of the α,β-unsaturated ketone of narasin.

Any suitable protecting group for carboxylic acids may be used. Advantageously, the carboxylic acid is protected in the form of an ester, such as a methyl ester, an allyl ester or a silyl ester. The C1 carboxyl of narasin may also be protected prior to the chemoselective oxidation to the α,β-unsaturated ketone.

Suitable protecting groups are for example disclosed in Greene, "Protective Groups In Organic Synthesis", (John Wiley & Sons, New York (1981)).

The protected α,β-unsaturated ketone of narasin may be represented by the following formula (C):

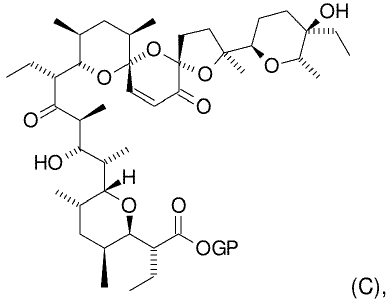

GP representing a protective group as defined above.

(c) Reacting the α,β-unsaturated ketone with an amine HNR¹R² and simultaneously or subsequently reducing the imine.

Methods for preparing amines by reductive amination are well known in the art. Advantageously, the imine is formed by reacting the amine in a polar solvent in the presence of an acid. In a particular embodiment, the imine is formed in a mixture of an alcohol, such as methanol or ethanol and acetic acid. The reduction of the imine into the amine is advantageously done with a borohydride, such as sodium borohydride or sodium cyanoborohydride, in the presence of a cerium salt, such as cerium trichloride CeCl₃.

The optionally protected C20-aminonarasin thus obtained may be represented by the following formula (D):

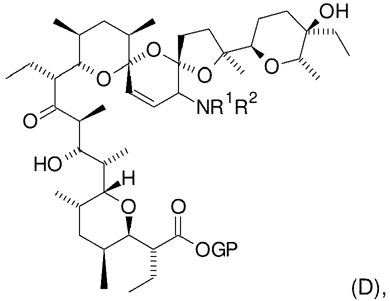

GP representing a protective group as defined above.

(d) Optionally deprotecting the C1 functionality of narasin to provide the carboxylic acid, and thus the compound (I) according to the invention.

Methods for the deprotection of an ester are for example disclosed in Greene, "Protective Groups In Organic Synthesis", (John Wiley & Sons, New York (1981)).

According to an embodiment, narasin is first oxidized by its reaction with an oxidation agent such as MnO₂, in particular in dichloromethane, preferably at room temperature, in order to obtain the compound C20-oxonarasin having the following formula (II):

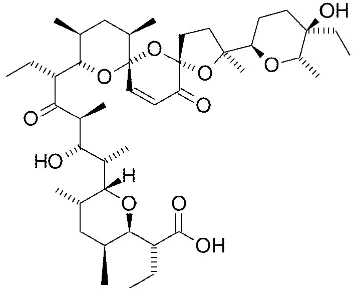

The compound of formula (II) is then reacted with an amine of formula HNR¹R², R¹ and R² being as defined above. This reaction is preferably carried out at room temperature, in particular in the presence of a solvent comprising preferably methanol and acetic acid.

The obtained mixture is then stirred and CeCl₃ x 7H₂O is added to said mixture, followed by the dropwise addition of a solution of NaBH₃CN in an alcoholic solvent, preferably in anhydrous methanol, at room temperature. Next, the reaction mixture is evaporated to dryness and CH₂Cl₂ is added thereto, and then the insoluble components are filtered off and the filtrate is purified in order to obtain the compound of formula (I).

### Compositions and uses

The present invention also relates to the compound as defined above, in particular having the formula (I), (I-1), (I-2) or (I-3), for use as drug.

The present invention also relates to a medicament comprising a compound as defined above, in particular having the formula (I), (I-1), (I-2) or (I-3), or a pharmaceutically acceptable salt thereof.

The present invention also relates to a pharmaceutical composition comprising, at least one compound as defined above, in particular having the formula (I), (I-1), (I-2) or (I-3), or a pharmaceutically acceptable salt thereof, and also at least one pharmaceutically acceptable excipient.

### Anti-cancer uses

The compounds of formula (I) of the invention may be used for preventing and/or treating cancer.

By "preventing", it is meant avoiding the cancer to occur.

By "treatment", it is meant the curative treatment of cancer. A curative treatment is defined as a treatment that completely treat (cure) or partially treat cancer (i.e. induces tumor growth stabilization, retardation or regression).

The "subject" refers to any subject and typically designates a patient, preferably a subject undergoing a treatment of cancer such as immunotherapy, chemotherapy and/or radiotherapy. In any case, the subject is preferably a vertebrate, more preferably a mammal, even more preferably a human being.

By "cancer", it is meant any type of cancer. The cancer may be solid or non-solid, and may be for example selected from a colon cancer, a colorectal cancer such as colorectal cancers with a BRAF mutation (especially BRAF V600E), a melanoma, a bone cancer, a breast cancer such as triple-negative breast cancer (i.e. breast cancer that tests negative for estrogen receptors, progesterone receptors, and excess HER2 protein), a thyroid cancer, a prostate cancer, an ovarian cancer, a lung cancer, a pancreatic cancer, a glioma such as a glioblastoma, a cervical cancer, an endometrial cancer, a head and neck cancer, a liver cancer, a bladder cancer, a renal cancer, a skin cancer, a stomach cancer, a testis cancer, an urothelial cancer or an adrenocortical carcinoma, leukemia such as acute myeloid leukemia, but also non-solid cancers such as lymphoma or multiple myeloma.

Preferably, the cancer is a colon cancer, a colorectal cancer such as colorectal cancers with a BRAF mutation (especially BRAF V600E), a breast cancer such as triple-negative breast cancer (i.e. breast cancer that tests negative for estrogen receptors, progesterone receptors, and excess HER2 protein), a pancreatic cancer, a glioma such as a glioblastoma, leukemia such as acute myeloid leukemia, lymphoma or multiple myeloma.

The cancer can be a metastatic cancer or not. A typical cancer is a cancer resistant to the first-line chemotherapy.

The present invention thus relates to a compound as defined above, in particular having the formula (I), (I-1), (I-2) or (I-3), or a pharmaceutically acceptable salt thereof, for use in preventing and/or treating cancer.

Preferably, the cancer is selected from the group consisting of: a colon cancer, a colorectal cancer, a melanoma, a bone cancer, a breast cancer, a thyroid cancer, a prostate cancer, an ovarian cancer, a lung cancer, a pancreatic cancer, a glioma, a cervical cancer, an endometrial cancer, a head and neck cancer, a liver cancer, a bladder cancer, a renal cancer, a skin cancer, a stomach cancer, a testis cancer, an urothelial cancer or an adrenocortical carcinoma, leukemia, lymphoma, and multiple myeloma.

The invention also relates to the use of at least one compound of formula (I) in increasing the sensitivity of a cancer to a chemotherapeutic drug.

A further object of the invention is the use of at least one compound of formula (I) in decreasing the resistance of a cancer with respect to a chemotherapeutic drug.

The invention also relates to a product comprising:
a) at least one compound of formula (I) of the invention, and
b) at least one additional therapeutic agent, as a combination product for a simultaneous, separate or sequential use in treating cancer, and/or in preventing cancer metastasis, and/or in preventing cancer recurrence, and/or in decreasing resistance to the additional therapy b), in a subject.

Also disclosed is the use of at least one compound of formula (I) of the invention, in preventing and/or treating a cancer in combination or in association with at least one additional therapeutic agent.

Also disclosed is the use of at least one compound of formula (I) of the invention, in preventing and/or treating a cancer in a subject treated by at least one additional therapeutic agent. The invention also relates to at least one compound of formula (I) of the invention, for use as an adjuvant cancer therapy. An adjuvant therapy is a therapy for treating cancer that is given besides a primary or initial therapy ("first-line therapy"), to maximize its effectiveness.

Said additional therapy b) may be immunotherapy, chemotherapy and/or radiotherapy. Preferably, the additional therapy b) is immunotherapy and/or chemotherapy.

By "immunotherapy" or "immunotherapeutic agent", it is meant a therapy which is able to induce, enhance or suppress an immune response. Said immunotherapy or immunotherapeutic agent is preferably chosen from cytokines, chemokines, growth factors, growth inhibitory factors, hormones, soluble receptors, decoy receptors; monoclonal or polyclonal antibodies, mono-specific, bi-specific or multi-specific antibodies, monobodies, polybodies; vaccines; or adoptive specific immunotherapy.

Preferably, the immunotherapy or immunotherapeutic agent is chosen from monoclonal or polyclonal antibodies, mono-specific, bi-specific or multi-specific antibodies, monobodies, polybodies, such as anti-angiogenic agents like Bevacuzimab (mAb, inhibiting VEGF-A, Genentech); IMC-1121B (mAb, inhibiting VEGFR-2, ImClone Systems); CDP-791 (Pegylated DiFab, VEGFR-2, Celltech); 2C3 (mAb, VEGF-A, Peregrine Pharmaceuticals); VEGF-trap (soluble hybrid receptor VEGF-A, PIGF (placenta growth factor) Aventis/Regeneron).

Preferably, the immunotherapy or immunotherapeutic agent is a monoclonal antibody, preferably an anti-checkpoint antibody.

The anti-checkpoint antibodies comprise antibodies directed against an immune checkpoint, which may be chosen from PD1, PDL1, PDL2, CTLA4, BTLA, CD27, CD40, OX40, GITR (also called "Tumor necrosis factor receptor superfamily member 18" or TNFRSF18), CD137 (also called 4-1BB or TNFRS9), CD28, ICOS, IDO (indoleamine 2,3-dioxygenase), B7H3 (also called CD276), KIR2DL2 (also called killer cell immunoglobulin-like receptor 2DL2), NKG2 (a family of the C-type lectin receptors), LAG3 (also called Lymphocyte Activation Gene-3) and CD70. Preferably the anti-checkpoint antibodies are anti-PD1, anti-PDL1, anti-PDL2 or anti-CTLA4 antibodies. Anti-PD1 antibodies include nivolumab and pembrolizumab. Anti-CTLA4 antibodies include ipilimumab and tremelimumab.

The "chemotherapy" or "chemotherapeutic agent" refers to compounds which are used in the treatment of cancer and that have the functional property of inhibiting a development or progression of a neoplasm in a human, particularly a malignant (cancerous) lesion.

Chemotherapeutic agents have different modes of actions, for example, by influencing either DNA or RNA and interfering with cell cycle replication.

Examples of chemotherapeutic agents that act at the DNA level or on the RNA level are:
- anti-metabolites, such as Azathioprine, Cytarabine, Fludarabine phosphate, Fludarabine, Gemcitabine, cytarabine, Cladribine, capecitabine 6-mercaptopurine, 6-thioguanine, methotrexate, 5-fluorouracil and hydroxyurea;
- alkylating agents, such as Melphalan, Busulfan, Cisplatin, Carboplatin, Cyclophosphamide, Ifosphamide, Dacarabazine, Fotemustine, Procarbazine, Chlorambucil, Thiotepa, Lomustine, Temozolomide;
- anti-mitotic agents, such as Vinorelbine, Vincristine, Vinblastine, Docetaxel, Paclitaxel;
- topoisomerase inhibitors, such as Doxorubicin, Amsacrine, Irinotecan, Daunorubicin, Epirubicin, Mitomycin, Mitoxantrone, Idarubicin, Teniposide, Etoposide, Topotecan;
- antibiotics, such as actinomycin and bleomycin;
- asparaginase;
- anthracyclines or taxanes.

Other chemotherapeutic agents are tyrosine kinase inhibitors (TKIs). A number of TKIs are in late and early stage development for treatment of various types of cancer. Exemplary TKIs include, but are not limited to: BAY 43-9006 (Sorafenib, Nexavar^{®}) and SU11248 (Sunitinib, Sutent^{®}), Imatinib mesylate (Gleevec^{®}, Novartis); Gefitinib (Iressa^{®}, AstraZeneca); Erlotinib hydrochloride (Tarceva^{®}, Genentech); Vandetanib (Zactima^{®}, AstraZeneca), Tipifarnib (Zarnestra^{®}, Janssen-Cilag); Dasatinib (Sprycel^{®}, Bristol Myers Squibb); Lonafarnib (Sarasar^{®}, Schering Plough); Vatalanib succinate (Novartis, Schering AG); Lapatinib (Tykerb^{®}, GlaxoSmithKline); Nilotinib (Novartis); Lestaurtinib (Cephalon); Pazopanib hydrochloride (GlaxoSmithKline); Axitinib (Pfizer); Canertinib dihydrochloride (Pfizer); Pelitinib (National Cancer Institute, Wyeth); Tandutinib (Millennium); Bosutinib (Wyeth); Semaxanib (Sugen, Taiho); AZD-2171 (AstraZeneca); VX-680 (Merck, Vertex); EXEL-0999 (Exelixis); ARRY-142886 (Array BioPharma, AstraZeneca); PD-0325901 (Pfizer); AMG-706 (Amgen); BIBF-1120 (Boehringer Ingelheim); SU-6668 (Taiho); CP-547632 (OSI); (AEE-788 (Novartis); BMS-582664 (Bristol-Myers Squibb); JNK-401 (Celgene); R-788 (Rigel); AZD-1152 HQPA (AstraZeneca); NM-3 (Genzyme Oncology); CP-868596 (Pfizer); BMS-599626 (Bristol-Myers Squibb); PTC-299 (PTC Therapeutics); ABT-869 (Abbott); EXEL-2880 (Exelixis); AG-024322 (Pfizer); XL-820 (Exelixis); OSI-930 (OSI); XL-184 (Exelixis); KRN-951 (Kirin Brewery); CP-724714 (OSI); E-7080 (Eisai); HKI-272 (Wyeth); CHIR-258 (Chiron); ZK-304709 (Schering AG); EXEL-7647 (Exelixis); BAY-57-9352 (Bayer); BIBW-2992 (Boehringer Ingelheim); AV-412 (AVEO); YN-968D1 (Advenchen Laboratories); Staurosporin, Midostaurin (PKC412, Novartis); Perifosine (AEterna Zentaris, Keryx, National Cancer Institute); AG-024322 (Pfizer); AZD-1152 (AstraZeneca); ON-01910Na (Onconova); and AZD-0530 (AstraZeneca).

Herein described are also (i) a method for preventing or treating cancer, (ii) a method for increasing the sensitivity of a cancer to a chemotherapeutic agent, and (iii) a method for decreasing the resistance of a cancer with respect to a chemotherapeutic drug, each of said methods comprising administering to a subject in need thereof with an effective amount of at least one compound of formula (I) as defined above, preferably together with a chemotherapeutic drug.

The compound of formula (I) of the invention is preferably administered at a therapeutically effective amount or dose. As used herein, "a therapeutically effective amount or dose" refers to an amount of the compound of the invention which prevents, removes, slows down the disease, or reduces or delays one or several symptoms or disorders caused by or associated with said disease in the subject, preferably a human being. The effective amount, and more generally the dosage regimen, of the compound of the invention and pharmaceutical compositions thereof may be determined and adapted by the one skilled in the art. An effective dose can be determined by the use of conventional techniques and by observing results obtained under analogous circumstances. The therapeutically effective dose of the compound of the invention will vary depending on the disease to be treated or prevented, its gravity, the route of administration, any co-therapy involved, the patient's age, weight, general medical condition, medical history, etc.

Typically, the amount of the compound to be administered to a patient may range from about 0.01 to 500 mg/kg of body weight for a human patient. In a particular embodiment, the pharmaceutical composition according to the invention comprises 0.01 mg/kg to 300 mg/kg of the compound of the invention, preferably from 0.01 mg/kg to 3 mg/kg, for instance from 25 to 300 mg/kg.

In a particular aspect, the compounds of the invention can be administered to the subject by parenteral route, topical route, oral route or intravenous injection. The compound or the nanoparticle of the invention may be administered to the subject daily (for example 1, 2, 3, 4, 5, 6 or 7 times a day) during several consecutive days, for example during 2 to 10 consecutive days, preferably from 3 to 6 consecutive days. Said treatment may be repeated during 1, 2, 3, 4, 5, 6 or 7 weeks, or every two or three weeks or every one, two or three months. Alternatively, several treatment cycles can be performed, optionally with a break period between two treatment cycles, for instance of 1, 2, 3, 4 or 5 weeks. The compound of the invention can for example be administered as a single dose once a week, once every two weeks, or once a month. The treatment may be repeated one or several times per year.

Doses are administered at appropriate intervals which can be determined by the skilled person. The amount chosen will depend on multiple factors, including the route of administration, duration of administration, time of administration, the elimination rate of the compound, or of the various products used in combination with said compound, the age, weight and physical condition of the patient and his/her medical history, and any other information known in medicine.

The administration route can be oral, topical or parenteral, typically rectal, sublingual, intranasal, intra-peritoneal (IP), intra-venous (IV), intra-arterial (IA), intramuscular (IM), intra-cerebellar, intrathecal, intratumoral and/or intradermal. The pharmaceutical composition is adapted for one or several of the above-mentioned routes. The pharmaceutical composition is preferably administered by injection or by intravenous infusion of suitable sterile solutions, or in the form of liquid or solid doses via the alimentary canal.

The present invention also relates to a composition comprising, in a pharmaceutically acceptable medium, at least one compound of formula (I) according to the invention. Such a composition comprises a pharmaceutically acceptable medium (or carrier).

The carrier must be "acceptable" in the sense of being compatible with the other ingredients of the formulations and not deleterious to the recipient thereof.

The pharmaceutical composition can be formulated as solutions in pharmaceutically compatible solvents or as gels, oils, emulsions, suspensions, or dispersions in suitable pharmaceutical solvents or vehicles, or as pills, tablets, capsules, powders, suppositories, etc. that contain solid vehicles in a way known in the art, possibly through dosage forms or devices providing sustained and/or delayed release. For this type of formulation, an agent such as cellulose, lipids, carbonates or starches are used advantageously.

Agents or vehicles that can be used in the formulations (liquid and/or injectable and/or solid) are excipients or inert vehicles, i.e. pharmaceutically inactive and non-toxic vehicles.

Mention may be made, for example, of saline, physiological, isotonic and/or buffered solutions, compatible with pharmaceutical use and known to those skilled in the art. The compositions may contain one or more agents or vehicles chosen from dispersants, solubilizers, stabilizers, preservatives, etc.

Particular examples are methylcellulose, hydroxymethylcellulose, carboxymethylcellulose, cyclodextrins, polysorbate 80, mannitol, gelatin, lactose, liposomes, vegetable oils or animal, acacia, etc. Preferably, vegetable oils are used.

Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion.

Formulations suitable for parenteral administration conveniently comprise a sterile oily or aqueous preparation of the active ingredient which is preferably isotonic with the blood of the recipient. Every such formulation can also contain other pharmaceutically compatible and non-toxic auxiliary agents, such as, e.g. stabilizers, antioxidants, binders, dyes, emulsifiers or flavoring substances.

The invention is now illustrated by the following examples.

This research was by funded by the European Research Council under the European Union's Horizon 2020 research and innovation programme grant agreement No 647973, the Foundation Charles Defforey-Institut de France, Ligue Contre le Cancer (Equipes Labellisées).

### EXAMPLES

### PREPARATION OF COMPOUNDS OF FORMULA (I)

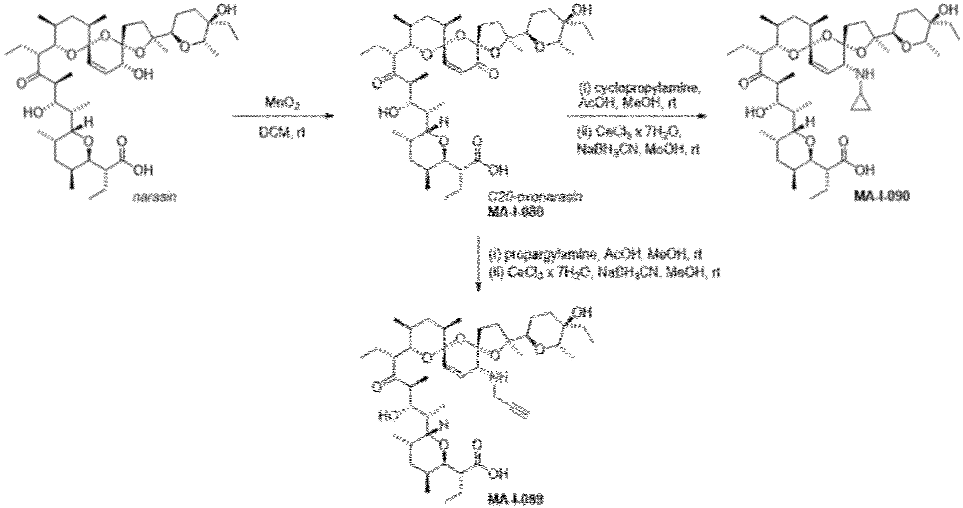

### Chemical modification of narasin.

### Synthesis of C20-oxonarasin MA-I-080

To a stirred solution of narasin (40 mg, 0.05 mmol, 1.0 equiv.) in CH₂Cl₂ (5 mL), activated MnO₂ (174 mg, 2.00 mmol, 40.0 equiv.) was added in one portion, and the black suspension was stirred at room temperature for 24 h. After that, the second portion of activated MnO₂ (174 mg, 2.00 mmol, 40.0 equiv.) was added, and the stirring was continued at room temperature for the next 24 h. The reaction mixture was then filtered through Celite, evaporated to dryness under reduced pressure, and the residue was purified using HPLC equipped with a C18-reverse phase column (gradient: ACN/H₂O/FA 4/1/0.1 to ACN/FA 1/0.1). Pure product of the reaction was quantitatively obtained as a white amorphous solid.

Yield: 39 mg, 98%. Isolated as a white amorphous solid, >95% pure by NMR, and a single spot by TLC; R_{f}: 0.44 in CH₂Cl₂/EtOAc 50%. UV-active and strain green with PMA; ¹H NMR (500 MHz, CD₂Cl₂) δ 7.16 (d, *J =* 10.8 Hz, 1H), 6.10 (d, *J =* 10.8 Hz, 1H), 4.12 (q, *J =* 6.1 Hz, 1H), 3.91 (d, *J =* 10.3 Hz, 1H), 3.86 (dd, *J =* 10.3, 5.3 Hz, 1H), 3.78 (d, *J =* 10.2 Hz, 1H), 3.51 (dd, *J* = 9.8, 1.7 Hz, 1H), 3.44 (t, *J =* 4.8 Hz, 1H), 3.21 (s, 1H), 2.85-2.69 (m, 2H), 2.59 (dd, *J =* 10.9, 1.4 Hz, 1H), 2.45 (td, *J =* 12.4, 8.9 Hz, 1H), 2.35-2.20 (m, 1H), 2.05-0.60 (m, 55H) ppm; ¹³C NMR (126 MHz, CD₂Cl₂) δ 214.5, 191.1, 177.5, 144.6, 126.4, 106.4, 98.1, 91.6, 78.7, 76.3, 75.3, 74.8, 73.1, 72.4, 68.9, 55.4, 49.7, 48.8, 40.4, 39.2, 36.7, 36.1, 35.8, 34.7, 33.1, 31.0, 29.5, 28.6, 25.0, 24.3, 23.9, 22.0, 19.1, 18.0, 16.13, 16.07, 14.0, 13.7, 13.0, 12.3, 12.2, 7.5, 6.9 ppm; HRMS (ESI⁺) m/z [M+NH₄]⁺ Calcd for C₄₃H₇₄NO₁₁ 780.5262, Found 780.5265.

### Example 1: Synthesis of C20-propargyloaminonarasin (compound 1)

Under argon atmosphere, C20-oxonarasin (**MA-I-080**) (67 mg, 0.09 mmol, 1.0 equiv.) was dissolved in anhydrous MeOH (4 mL), followed by the addition of molecular sieves 4Å, propargylamine (55 µL, 0.87 mmol, 10.0 equiv.) and glacial acetic acid (35 µL). The mixture was stirred 3 h at room temperature, prior to the addition of CeCl₃ x 7H₂O (32 mg, 0.09 mmol, 1.0 equiv.), and the dropwise addition of a solution of NaBH₃CN (7.0 mg, 0.12 mmol, 1.3 equiv.) in anhydrous MeOH (3 mL) over 8 h at room temperature, using a syringe pump. Then, the yellow reaction mixture was evaporated to dryness. A small portion of CH₂Cl₂ was added to the oily residue, and the insoluble components were filtered off. The filtrate was evaporated *in vacuo* and purified using HPLC equipped with a C18-reverse phase column (gradient: ACN/H₂O/FA 1/1/0.1 to ACN/FA 1/0.1). Pure product of the reaction was obtained as a white amorphous solid with 39% yield.

Yield: 27 mg, 39%. Isolated as a white amorphous solid, >95% pure by NMR, and a single spot by TLC; R_{f}: 0.78 in CH₂Cl₂/EtOAc 50%. Strain green with PMA; ¹H NMR (400 MHz, CD₂Cl₂) δ 6.37 (dd, *J =* 10.0, 5.3 Hz, 1H), 6.20 (d, *J =* 10.2 Hz, 1H), 4.24 (d, *J* = 9.8 Hz, 1H), 4.16 (dt, *J* = 16.0, 2.1 Hz, 1H), 3.89-3.79 (m, 2H), 3.78-3.70 (m, 2H), 3.59 (d, *J* = 5.4 Hz, 1H), 3.57-3.50 (m, 2H), 2.67-2.54 (m, 3H), 2.29 (t, *J =* 2.3 Hz, 1H), 2.25-2.14 (m, 1H), 2.10-2.00 (m, 1H), 1.99-1.83 (m, 4H), 1.80-0.60 (m, 53H) ppm; ¹³C NMR (101 MHz, CD₂Cl₂) δ 215.0, 178.7, 130.6, 128.0, 109.0, 99.7, 88.7, 80.3, 78.2, 77.6, 75.7, 73.8, 73.5, 73.4, 71.2, 69.2, 55.9, 53.2, 50.4, 50.3, 40.0, 39.5, 38.5, 36.5, 36.1, 35.6, 32.8, 31.9, 30.9, 29.6, 29.2, 28.8, 24.4 (2C), 21.8, 17.9, 17.6, 16.5, 15.8, 14.6, 13.5, 13.2, 12.9, 12.4, 8.2, 6.6 ppm; HRMS (ESI⁺) m/z [M+H]⁺ Calcd for C₄₆H₇₆NO₁₀ 802.5469, Found 802.5469.

### Example 2: Synthesis of C20-cyclopropyloaminonarasin (compound 2)

Under argon atmosphere, C20-oxonarasin (**MA-I-080**) (88 mg, 0.11 mmol, 1.0 equiv.) was dissolved in anhydrous MeOH (5 mL), followed by the addition of molecular sieves 4Å, cyclopropylamine (79 µL, 1.11 mmol, 10.0 equiv.) and glacial acetic acid (45 µL). The mixture was stirred 3 h at room temperature, prior to the addition of CeCl₃ x 7H₂O (42 mg, 0.11 mmol, 1.0 equiv.), and the dropwise addition of a solution of NaBH₃CN (9.0 mg, 0.16 mmol, 1.4 equiv.) in anhydrous MeOH (4 mL) over 8 h at room temperature, using a syringe pump. After that, the cloudy reaction mixture was filtered, and the filtrate was evaporated to dryness. The oily residue was dissolved in a small portion of CH₂Cl₂, the insoluble components were filtered off, the filtrate was evaporated *in vacuo* and purified using HPLC equipped with a C18-reverse phase column (gradient: ACN/H₂O/FA 1/1/0.1 to ACN/FA 1/0.1). Pure product of the reaction was obtained as a white amorphous solid with 47% yield.

Yield: 44 mg, 47%. Isolated as a white amorphous solid, >95% pure by NMR, and a single spot by TLC; R_{f}: 0.87 in CH₂Cl₂/EtOAc 50%. Strain green with PMA; ¹H NMR (500 MHz, CD₂Cl₂) δ 6.43 (dd, *J =* 10.3, 4.7 Hz, 1H), 6.17 (d, *J =* 10.3 Hz, 1H), 4.20 (dd, *J* = 10.0, 1.3 Hz, 1H), 3.83-3.75 (m, 2H), 3.73 (q, *J =* 6.8 Hz, 1H), 3.56 (ddd, *J* = 13.8, 10.2, 2.9 Hz, 2H), 3.36 (d, *J =* 4.7 Hz, 1H), 2.80 (ddd, *J =* 11.0, 7.4, 3.8 Hz, 1H), 2.64 (dq, *J* = 9.9, 7.1 Hz, 1H), 2.57 (ddd, *J =* 9.3, 7.7, 5.0 Hz, 2H), 2.22-2.12 (m, 1H), 2.09-1.86 (m, 5H), 1.80-0.65 (m, 55H), 0.62-0.55 (m, 1H), 0.52-0.45 (m, 1H) ppm; ¹³C NMR (126 MHz, CD₂Cl₂) δ 215.0, 178.6, 130.8, 126.8, 108.2, 99.9, 89.1, 78.1, 77.4, 76.0, 73.7, 71.2, 69.4, 56.9, 56.0, 50.4 (2C), 40.3, 39.4, 38.7, 36.1, 35.6, 32.9, 31.3, 30.9, 30.6, 29.5, 29.2, 28.8, 25.0, 24.6, 22.2, 17.9, 17.7, 16.6, 15.9, 14.5, 13.6, 13.4, 13.1, 12.5, 8.2, 6.6, 6.2, 5.1 ppm, one signal overlapped; HRMS (ESI⁺) m/z [M+H]⁺ Calcd for C₄₆H₇₈NO₁₀ 804.5626, Found 804.5626.

## Claims

1. A compound having the formula (I): 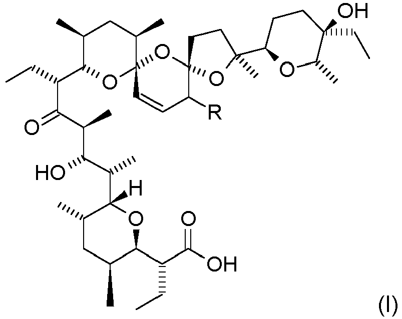
wherein R is a group -NR¹R², R¹ and R² being identical or different, and independently chosen from the group consisting of: H, a (C₁-C₂₀)alkyl group, a (C₃-C₆)cycloalkyl group, a (C₂-C₂₀)alkynyl group, and a (C₆-C₁₀)aryl(C₁-C₆)alkyl group,
as well as its epimers.

2. The compound of claim 1, having the formula (I-1) or (I-2): 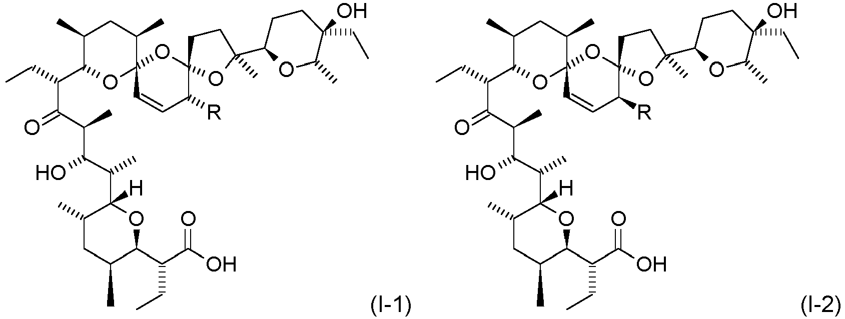 R being as defined in claim 1.

3. The compound of claim 1 or 2, wherein R is a group -NHR², R² being selected from the group consisting of: a (C₁-C₂₀)alkyl group, a (C₃-C₆)cycloalkyl group, a (C₂-C₂₀)alkynyl group, and a (C₆-C₁₀)aryl(C₁-C₆)alkyl group.

4. The compound of claim 3, wherein R² is a (C₃-C₆)cycloalkyl group or a (C₂-C₂₀)alkynyl group.

5. The compound of claim 3 or 4, wherein R² is a cyclopropyl or a propynyl group.

6. The compound of any one of the preceding claims, having the formula (I-3): 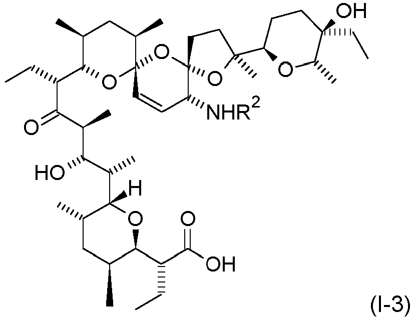 R² being a (C₃-C₆)cycloalkyl group or a (C₂-C₂₀)alkynyl group.

7. The compound of any one of the preceding claims, being chosen from the following compounds:

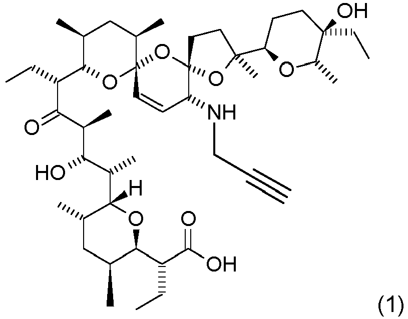

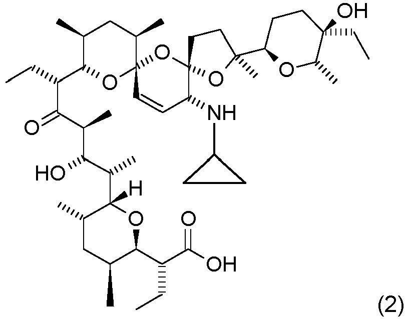

8. A process for the preparation of a compound of formula (I) according to any one of claims 1 to 7, comprising the following steps:
- a step of oxidizing the C20 allylic hydroxyl of narasin to the corresponding α,β-unsaturated ketone; and
- a step of reacting the α,β-unsaturated ketone with an amine HNR¹R², in order to obtain the corresponding imine, and a step for simultaneously or subsequently reducing the imine, in order to obtain the compound of formula (I).

9. The compound according to any one of claims 1 to 7, for use as drug.

10. A medicament comprising a compound according to any one of claims 1 to 7, or a pharmaceutically acceptable salt thereof.

11. A pharmaceutical composition comprising, at least one compound according to any one of claims 1 to 7, or a pharmaceutically acceptable salt thereof, and also at least one pharmaceutically acceptable excipient.

12. The compound of any one of claims 1 to 7, for use in preventing and/or treating cancer.

13. The compound for use according to claim 12, wherein the cancer is selected from the group consisting of: a colon cancer, a colorectal cancer, a melanoma, a bone cancer, a breast cancer, a thyroid cancer, a prostate cancer, an ovarian cancer, a lung cancer, a pancreatic cancer, a glioma, a cervical cancer, an endometrial cancer, a head and neck cancer, a liver cancer, a bladder cancer, a renal cancer, a skin cancer, a stomach cancer, a testis cancer, an urothelial cancer or an adrenocortical carcinoma, leukemia, lymphoma, and multiple myeloma.

14. The compound of any one of claims 1 to 7, for use in preventing cancer metastasis and/or in preventing cancer recurrence and/or in decreasing resistance to a chemotherapy in a subject.

15. The compound for use according to claim 14, wherein the cancer is selected from solid and non-solid cancers.

16. The compound for use according to claim 15, wherein the cancer is selected from a colon cancer, a colorectal cancer such as colorectal cancers with a BRAF mutation especially BRAF V600E, a melanoma, a bone cancer, a breast cancer such as triple-negative breast cancer, a thyroid cancer, a prostate cancer, an ovarian cancer, a lung cancer, a pancreatic cancer, a glioma such as a glioblastoma, a cervical cancer, an endometrial cancer, a head and neck cancer, a liver cancer, a bladder cancer, a renal cancer, a skin cancer, a stomach cancer, a testis cancer, an urothelial cancer or an adrenocortical carcinoma, leukemia such as acute myeloid leukemia, lymphoma and multiple myeloma.

17. A product comprising:
a) a compound according to any one of claims 1 to 7, and
b) at least one immunotherapeutic agent chosen from cytokines, chemokines, growth factors, growth inhibitory factors, hormones, soluble receptors, decoy receptors, monoclonal or polyclonal antibodies, mono-specific, bispecific or multi-specific antibodies, monobodies, polybodies or vaccines; and/or at least one chemotherapeutic agent, as combination product for a simultaneous, separate or sequential use in treating cancer, and/or in preventing cancer metastasis, and/or in preventing cancer recurrence, and/or in decreasing resistance to the additional therapy b), in a subject.

18. A product for use as claim 17, wherein said subject being a human suffering from a cancer and resistant to chemotherapy.

## Patentansprüche

1. Verbindung der Formel (I): 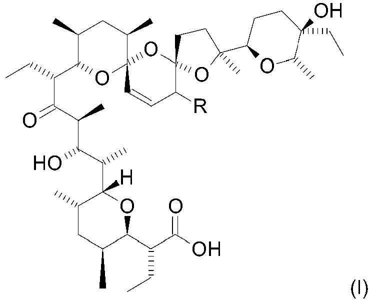 wobei R eine Gruppe -NR¹ R² ist, wobei R¹ und R² gleich oder verschieden sind und unabhängig voneinander aus der Gruppe ausgewählt sind, bestehend aus: H, einer (C₁ - C₂₀ )Alkylgruppe, einer (C₃-C₆)-Cycloalkylgruppe, einer (C₂-C₂₀)-Alkinylgruppe und einer (C₆-C₁₀)-Aryl(C₁-C₆)-alkylgruppe,
sowie deren Epimere.

2. Die Verbindung nach Anspruch 1 mit der Formel (1-1) oder (I-2): 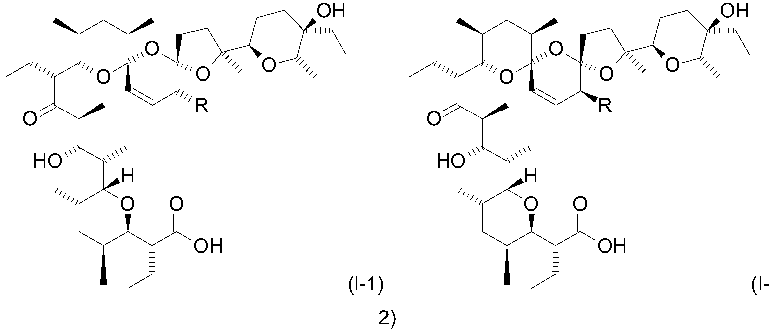 wobei R wie in Anspruch 1 definiert ist.

3. Die Verbindung nach Anspruch 1 oder 2, wobei R eine Gruppe -NHR² ist, wobei R² aus der Gruppe ausgewählt ist, bestehend aus: einer (C₁ - C₂₀)-Alkylgruppe, einer (C₃-C₆)-Cycloalkylgruppe, einer (C₂-C₂₀)-Alkinylgruppe und einer (C₆-C₁₀)-Aryl(C₁-C₆)-alkylgruppe.

4. Verbindung nach Anspruch 3, wobei R² eine (C₃ -C₆)-Cycloalkylgruppe oder eine (C₂ -C₂₀)-Alkinylgruppe ist.

5. Verbindung nach Anspruch 3 oder 4, wobei R² eine Cyclopropylgruppe oder eine Propinylgruppe ist.

6. Verbindung nach einem der vorstehenden Ansprüche mit der Formel (I-3): 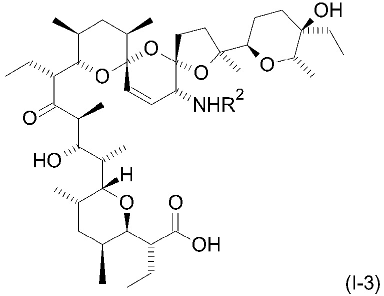 wobei R² eine (C₃-C₆)-Cycloalkylgruppe oder eine (C₂-C₂₀)-Alkinylgruppe ist.

7. Die Verbindung nach einem der vorstehenden Ansprüche, ausgewählt aus den folgenden Verbindungen:

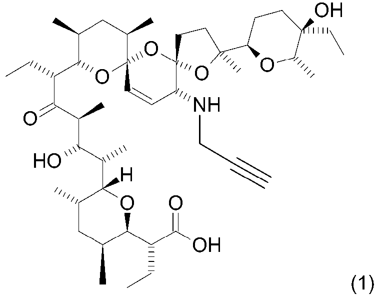

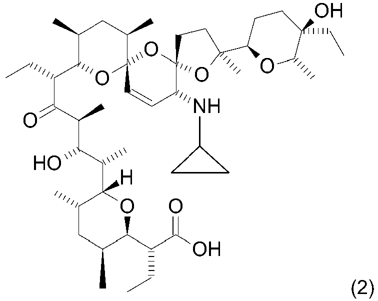

8. Verfahren zur Herstellung einer Verbindung der Formel (I) gemäß einem der Ansprüche 1 bis 7, umfassend die folgenden Schritte:
- einen Schritt der Oxidation der allylischen C20-Hydroxylgruppe von Narasin zum entsprechenden α,β-ungesättigten Keton; und
- einen Schritt, bei dem das α,β-ungesättigte Keton mit einem Amin HNR¹R² umgesetzt wird, um das entsprechende Imin zu erhalten, sowie einen Schritt der gleichzeitigen oder anschließenden Reduktion des Imins, um die Verbindung der Formel (I) zu erhalten.

9. Die Verbindung gemäß einem der Ansprüche 1 bis 7 zur Verwendung als Arzneimittel.

10. Arzneimittel, das eine Verbindung gemäß einem der Ansprüche 1 bis 7 oder ein pharmazeutisch verträgliches Salz davon enthält.

11. Pharmazeutische Zusammensetzung, umfassend mindestens eine Verbindung gemäß einem der Ansprüche 1 bis 7 oder ein pharmazeutisch verträgliches Salz davon sowie mindestens einen pharmazeutisch verträglichen Hilfsstoff.

12. Die Verbindung gemäß einem der Ansprüche 1 bis 7 zur Verwendung zur Vorbeugung und/oder Behandlung von Krebs.

13. Die Verbindung zur Verwendung gemäß Anspruch 12, wobei der Krebs aus der Gruppe ausgewählt ist, bestehend aus: Dickdarmkrebs, kolorektalem Karzinom, Melanom, Knochenkrebs, Brustkrebs, Schilddrüsenkrebs, Prostatakrebs, Eierstockkrebs, Lungenkrebs, Bauchspeicheldrüsenkrebs, Gliom, Gebärmutterhalskrebs, Endometriumkarzinom, Kopf-Hals-Krebs, Leberkrebs, Blasenkrebs, Nierenkrebs, Hautkrebs, Magenkrebs, Hodenkrebs, Urothelkarzinom oder Nebennierenrindenkarzinom, Leukämie, Lymphom und Multiplem Myelom.

14. Die Verbindung nach einem der Ansprüche 1 bis 7 zur Verwendung zur Verhinderung von Krebsmetastasen und/oder zur Verhinderung eines Krebsrezidivs und/oder zur Verringerung der Resistenz gegen eine Chemotherapie bei einem Patienten.

15. Die Verbindung zur Verwendung gemäß Anspruch 14, wobei der Krebs aus soliden und nicht-soliden Krebserkrankungen ausgewählt ist.

16. Die Verbindung zur Verwendung gemäß Anspruch 15, wobei der Krebs ausgewählt ist aus: Dickdarmkrebs, kolorektalem Karzinom, wie beispielsweise kolorektalen Karzinomen mit einer BRAF-Mutation, insbesondere BRAF V600E, Melanom, Knochenkrebs, Brustkrebs, wie beispielsweise dreifach-negativem Brustkrebs, Schilddrüsenkrebs, Prostatakrebs, Eierstockkrebs, Lungenkrebs, Bauchspeicheldrüsenkrebs, Gliom, wie beispielsweise Glioblastom, Gebärmutterhalskrebs, Endometriumkarzinom, Kopf-Hals-Krebs, Leberkrebs, Blasenkrebs, Nierenkrebs, Hautkrebs, Magenkrebs, Hodenkrebs, Urothelkarzinom oder Nebennierenrindenkarzinom, Leukämie, wie beispielsweise akuter myeloischer Leukämie, Lymphom und Multiplem Myelom.

17. Ein Produkt, umfassend:
a) eine Verbindung gemäß einem der Ansprüche 1 bis 7, und
b) mindestens ein immuntherapeutisches Mittel, ausgewählt aus Zytokinen, Chemokinen, Wachstumsfaktoren, wachstumshemmenden Faktoren, Hormonen, löslichen Rezeptoren, Köderrezeptoren; monoklonale oder polyklonale Antikörper, monospezifische, bispezifische oder multispezifische Antikörper, Monobodies, Polybodies oder Impfstoffe; und/oder mindestens ein Chemotherapeutikum,
als Kombinationspräparat zur gleichzeitigen, getrennten oder aufeinanderfolgenden Anwendung zur Behandlung von Krebs und/oder zur Verhinderung von Krebsmetastasen und/oder zur Verhinderung eines Krebsrezidivs und/oder zur Verringerung der Resistenz gegenüber der zusätzlichen Therapie b) bei einem Patienten.

18. Ein Produkt zur Verwendung gemäß Anspruch 17, wobei der Patient ein an Krebs leidender Mensch ist und gegenüber einer Chemotherapie resistent ist.

## Revendications

1. Composé de formule (I) : 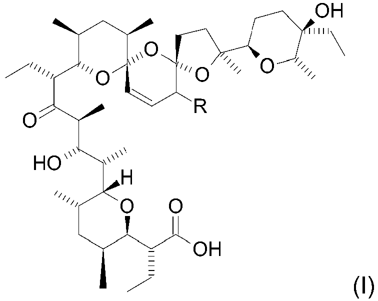
dans lequel R représente un groupe -NR¹R², R¹ et R² étant identiques ou différents, et choisis indépendamment parmi le groupe constitué de : H, un groupe alkyle en (C₁-C₂₀), un groupe cycloalkyle en (C₃-C₆), un groupe alcynyle en (C₂-C₂₀), et un groupe aryl(C₆-C₁₀)-alkyle(C₁-C₆),
ainsi que ses épimères.

2. Composé selon la revendication 1, de formule (I-1) ou (I-2) : 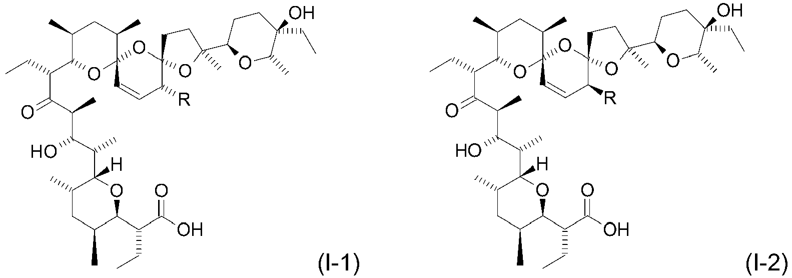 R étant tel que défini dans la revendication 1.

3. Composé selon la revendication 1 ou 2, dans lequel R représente un groupe -NHR², R² étant choisi parmi le groupe constitué de : un groupe alkyle en (C₁-C₂₀), un groupe cycloalkyle en (C₃-C₆), un groupe alcynyle en (C₂-C₂₀), et un groupe aryl(C₆-C₁₀)-alkyle(C₁ -C₆).

4. Composé selon la revendication 3, dans lequel R² est un groupe cycloalkyle en (C₃-C₆) ou un groupe alcynyle en (C₂-C₂₀).

5. Composé selon la revendication 3 ou 4, dans lequel R² est un groupe cyclopropyle ou propynyle.

6. Composé selon l'une quelconque des revendications précédentes, de formule (I-3) : 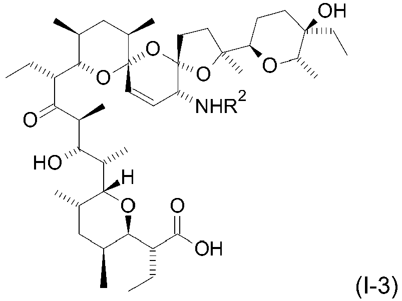 R² étant un groupe cycloalkyle en (C₃-C₆) ou un groupe alcynyle en (C₂-C₂₀).

7. Composé selon l'une quelconque des revendications précédentes, choisi parmi les composés suivants :

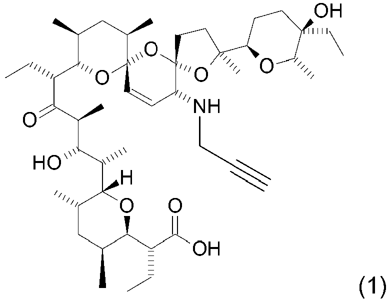

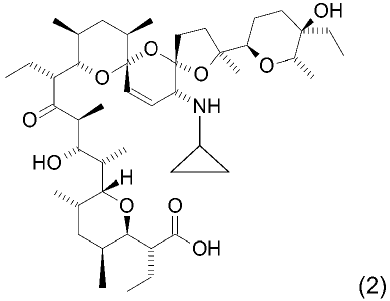

8. Procédé de préparation d'un composé de formule (I) selon l'une quelconque des revendications 1 à 7, comprenant les étapes suivantes :
- une étape d'oxydation du groupe hydroxyle allylique en C20 de la narasine en la cétone α,β-insaturée correspondante ; et
- une étape de réaction la cétone α,β-insaturée avec une amine HNR¹R², afin d'obtenir l'imine correspondante, et une étape pour réduire l'imine simultanément ou ultérieurement, afin d'obtenir le composé de formule (I).

9. Composé selon l'une quelconque des revendications 1 à 7, pour son utilisation comme médicament.

10. Médicament comprenant un composé selon l'une quelconque des revendications 1 à 7, ou un de ses sels pharmaceutiquement acceptables.

11. Composition pharmaceutique comprenant au moins un composé selon l'une quelconque des revendications 1 à 7, ou un de ses sels pharmaceutiquement acceptables, ainsi qu'au moins un excipient pharmaceutiquement acceptable.

12. Composé selon l'une quelconque des revendications 1 à 7, pour son utilisation dans la prévention et/ou le traitement du cancer.

13. Composé pour son utilisation selon la revendication 12, dans lequel le cancer est choisi parmi le groupe constitué par : un cancer du côlon, un cancer colorectal, un mélanome, un cancer des os, un cancer du sein, un cancer de la thyroïde, un cancer de la prostate, un cancer de l'ovaire, un cancer du poumon, un cancer du pancréas, un gliome, un cancer du col de l'utérus, un cancer de l'endomètre, un cancer de la tête et du cou, un cancer du foie, un cancer de la vessie, un cancer du rein, un cancer de la peau, un cancer de l'estomac, un cancer des testicules, un cancer urothélial ou un carcinome corticosurrénalien, une leucémie, un lymphome et un myélome multiple.

14. Composé selon l'une quelconque des revendications 1 à 7, pour son utilisation dans la prévention des métastases cancéreuses et/ou dans la prévention de la récidive d'un cancer et/ou dans la réduction de la résistance à une chimiothérapie chez un sujet.

15. Composé pour son utilisation selon la revendication 14, dans lequel le cancer est choisi parmi les cancers solides et non-solides.

16. Composé pour son utilisation selon la revendication 15, dans lequel est choisi parmi un cancer du côlon, un cancer colorectal tel que les cancers colorectaux présentant une mutation du gène BRAF, en particulier la mutation V600E du gène BRAF, un mélanome, un cancer des os, un cancer du sein tel qu'un cancer du sein triple négatif, un cancer de la thyroïde, un cancer de la prostate, un cancer de l'ovaire, un cancer du poumon, un cancer du pancréas, un gliome tel qu'un glioblastome, un cancer du col de l'utérus, un cancer de l'endomètre, un cancer de la tête et du cou, un cancer du foie, un cancer de la vessie, un cancer du rein, un cancer de la peau, un cancer de l'estomac, un cancer des testicules, un cancer urothélial ou un carcinome corticosurrénalien, une leucémie telle qu'une leucémie myéloïde aiguë, un lymphome et un myélome multiple.

17. Produit comprenant :
a) un composé selon l'une quelconque des revendications 1 à 7, et
b) au moins un agent immunothérapeutique choisi parmi les cytokines, les chimiokines, les facteurs de croissance, les facteurs inhibiteurs de croissance, les hormones, les récepteurs solubles, les récepteurs leurres, les anticorps monoclonaux ou polyclonaux, les anticorps monospécifiques, bispécifiques ou multispécifiques, les monocorps, les polycorps ou les vaccins ; et/ou au moins un agent chimiothérapeutique,
en tant que produit combiné pour une utilisation simultanée, séparée ou séquentielle dans le traitement du cancer, et/ou dans la prévention des métastases cancéreuses, et/ou dans la prévention de la récidive du cancer, et/ou dans la réduction de la résistance à la thérapie additionnelle b), chez un sujet.

18. Produit pour son utilisation selon la revendication 17, dans lequel ledit sujet est un être humain atteint d'un cancer et résistant à la chimiothérapie.
